# EUROPÄISCHE PATENTSCHRIFT

(11) **EP 0 799 605 B1**
(45) Veröffentlichungstag und Bekanntmachung des Hinweises auf die Patenterteilung: **01.06.2005**
(21) Anmeldenummer: 97250093.8
(22) Anmeldetag: 22.03.1997
(51) Int. Cl.: A61C 8/00

(54) **Kieferchirugisches Instrument (Osteotom) zur Schaffung von Öffnungen im Kieferknochen zum Einsetzen von Implantaten und seine Verwendung sowie Verfahren zur Schaffung von Öffnungen im Kieferknochen zum Einsetzen von Implantaten**
Dental surgical instrument for making holes in the jaw for the placing of implants, and a method for creating said holes
Instrument chirurgical dentaire pour la réalisation de cavités dans la mâchoire pour la pose d'implants, ainsi que méthode pour la réalisation de ces cavités

(30) Priorität: 01.04.1996 DE 19613743
(43) Veröffentlichungstag der Anmeldung: 08.10.1997
(73) Patentinhaber: FRIADENT GmbH, 68229 Mannheim (DE)
(72) Erfinder: Nowak, Marcus Dr., 14195 Berlin (DE)
(74) Vertreter: Lüke, Dierck-Wilm, Dipl.-Ing.

(56) Entgegenhaltungen:
- WO-A-95/21590
- DE-A- 2 619 650
- DE-A- 4 316 955
- ROBERT B. SUMMERS: "The Osteotome Technique: Part 2- The Ridge Expansion Osteotomy (REO) Procedure" COMPEND CONTIN EDUC DENT, Bd. XV, Nr. 4, 1994, Seite 422-434 XP002118818
- ROBERT B. SUMMERS: "A New Concept in Maxillary Implant Surgery: The Osteotome Technique" COMPEND ENTIN EDUC DENT, Bd. XV, Nr. 2, 1994, Seite 152-160 XP002118819

## Beschreibung

Die Erfindung bezieht sich auf einen Satz von kieferchirurgischen Instrumenten (Osteotome) aus Halter, Schaft und im Querschnitt kreisförmiger Arbeitsspitze zur Schaffung von Öffnungen im Kieferknochen zum Einsetzen von Implantaten.

Ein kieferchirurgisches Instrument (Osteotom) ist aus der DE 43 16 955 A1 vorbekannt. Dieses dient zur operativen Aufspaltung und Spreizung von Kieferabschnitten zum Einsetzen von im Querschnitt kreisförmigen Implantaten, insbesondere im Oberkiefer. Nachteilig hierbei ist wegen der Schlitzform der geschaffenen Öffnung im Kiefer eine relativ große Länge der Osteotomielinie. Dies führt häufig zu postoperativen Lockerungen des Implantates oder zu einer schlechten Wundheilung. Dies soll mit der besser am Kieferkamm ansetzbaren Arbeitsspitze des vorbekannten Dentalinstrumentes vermieden werden, jedoch ist ein relativ großer operativer Aufwand bei Anwendung des vorbekannten Dentalinstrumentes notwendig, um ein Implantat zur Befestigung von Zahnkronen einzusetzen.

Ein Satz von kieferchirurgischen Instrumenten der gattungsgemäßen Art ist aus der DE 26 19 650 vorbekannt. Es sind hier in Fig. 10 und 11 ein Vorbohrer bzw. ein Fertigbohrer dargestellt, mit denen die Öffnungen zum Einsetzen von Implantaten durch Bohren erzeugt werden. Hierbei wird aber wertvolles Knochenmaterial abgetragen, wodurch die Sitzfestigkeit des Implantates nicht gewährleistet ist. Es ist in der Zahnmedizin bekannt, daß das Knochenmaterial des Oberkiefers wesentlich weicher ist als das Knochenmaterial des Unterkiefers, wobei das Knochenmaterial des Oberkiefers in seiner Festigkeit mit Balsaholz verglichen werden kann. Das Knochenmaterial wird in mehrere Knochenklassen unterteilt, wobei auch Osteoporose, d.h die Strukturveränderung des Knochenmaterials im Alter, berücksichtigt wird. Insbesondere im Hinblick auf die Weichheit des Knochenmaterials des Oberkiefers erweist sich das Bohren zur Bildung von Öffnungen im Oberkiefer zum Einsetzen von Implantaten als äußerst ungünstig, da von dem ohnehin weichen Knochenmaterial noch Knochenmaterial entfernt wird.

Insbesondere zur Bildung von Öffnungen im Oberkiefer zum Einsetzen von Implantaten ist ein Instrument (Osteotom) nach Summers XPOO 2 118 818 vorbekannt, das aus einem stielartigen Halter und einem Schaft mit einer im Querschnitt kreisförmigen Arbeitsspitze gebildet ist und das auf seinem Schaft im unmittelbaren Anschluß an die Arbeitsspitze mit Skalierungsringen sowie auf seinem Ende mit einer konkaven Kalotte versehen ist. Die Summers-Osteotome haben die Form eines sich verstärkenden Rundstabes. Sie gibt es in sechs Größen mit steigenden Durchmessern. In die gebildete Kieferöffnung wird jeweils das nächst größere Instrument eingesetzt. Das Implantatbett wird durch Expansion und Kompression des Knochenmaterials geschaffen. Das Arbeitsende der Instrumente ist konkav und scharfrandig begrenzt. Durch Markierungen erfolgt eine Ermittlung der Eindringtiefe der Instrumente. Beim Eindrücken des Dentalinstrumentes zur Bildung der Öffnung wird das Knochenmaterial des Oberkiefers verdrängt und verfestigt. Das Knochenmaterial entspannt sich nach dem Herausziehen des Dentalinstrumentes und dem Einsetzen des Implantates und hält dieses fest. Es bildet sich in der Folgezeit neues Knochenmaterial um das eingesetzte Implantat herum. Somit wird bei dieser Implantat-Methode kein Knochenmaterial entfernt, sondern das Knochenmaterial, insbesondere des weichen Oberkiefers, wird im Gegenteil geradezu verfestigt. Nachteilig hierbei ist jedoch die Ausbildung von einfachen leicht konischen Höhlungen im Kiefer, insbesondere Oberkiefer, die kein sicheres Lager zur Befestigung insbesondere von Schraubenimplantaten gewährleisten. Insbesondere, da die bekannten Implantate unterschiedliche Außenkonturen haben, wie z.B. Stufenzylinderimplantate mit und ohne Schraubgewinde sowie Zylinderimplantate ohne und mit teilweisem oder durchgehenden Schraubengewinde, können die herkömmlichen, leicht konischen Instrumente nach Summers keine Gewähr dafür bieten, daß die gebildete, leicht konische Öffnung im Kiefer das eingesetzte Implantat sicher hält.

Der Erfindung liegt von daher die Aufgabe zugrunde, einen Satz kieferchirurgischer Instrumente (Osteotome) der gattungsgemäßen Art zu schaffen, mit welchem Öffnungen im Kiefer, insbesondere Oberkiefer, zum Einsetzen von Implantaten gebildet werden können, so daß diese mit großer Sitzfestigkeit in die Öffnungen im Kiefer einsetzbar sind.

Die Lösung der Aufgabe ergibt sich aus den kennzeichnenden Merkmalen des Patentanspruches 1. Erfindungsgemäß wird mit dem Vorformer mit konischer Arbeitsspitze, deren vorderes Ende spitzkegelig mit einer Spitze ausgeformt ist, zunächst eine kleinere Öffnung im Kiefer, insbesondere Oberkiefer, durch seitliches Wegdrücken des Knochenmaterials geschaffen, welche Öffnung noch nicht ausreichend ist, um das einzusetzende Implantat einzubringen.

Mittels des Implantatbettformers, dessen Arbeitsspitze entweder am vorderen Ende spitzkegelig oder flach abgerundet ausgebildet ist und eine zum freien Ende im Durchmesser abgestufte Außenkontur oder eine zylindrische oder eine konische Aussenkontur aufweist, wobei der Durchmesser der Arbeitsspitze des Vorformers etwas kleiner ist als der größte Durchmesser der Arbeitsspitze des Implantabettformers, wird das Knochenmaterial des Kiefers, insbesondere Oberkiefers, weiterhin soweit verdrängt, daß die im Kiefer geschaffene Öffnung bereits an die Außenkontur des einzusetzenden Implantats angepaßt ist. Dieses kann anschließend in die so geschaffene Öffnung im Kiefer, insbesondere Oberkiefer, eingedrückt bzw. eingedreht werden, wobei das Implantat durch das sich nach dem Herausziehen des Implantatbettformers und Einsetzen des Implantats wieder entspannende Knochenmaterial des Kiefers, insbesondere Oberkiefer, festgehalten wird. Das sich in der Folgezeit um das Implantat herum bildende neue Knochenmaterial hält das Implantat in der an dessen Außenkontur angepaßten Kieferöffnung optimal fest.

Erfindungsgemäß wird bei der primären Gestaltung des Implantatbettes der Kieferöffnung im Knochenmaterial bereits die spezifische Implantatform berücksichtigt, da mit dem zuletzt benutzten Implantatbettformer exakt die Form des Implantates in der Kieferöffnung geschaffen wird. Damit können auch Implantate mit stufenförmiger Kontur verwendet werden. Für diese Art von Implantatbett waren bisher rotierende Instrumente erforderlich.

Zum Einsetzen eines im Durchmesser abgestuften Implantats in Form eines Stufenzylinders oder einer Stufenschraube weist die Arbeitsspitze des Implantatbettformers eine zum freien Ende im Durchmesser abgestufte Außenkontur auf, die an die Außenkontur des Stufenzylinders angepaßt ist, d.h. dieser entspricht, wobei bei einer Stufenschraube der jeweilige Innendurchmesser des abgestuften Implantats für die Ausbildung der Form der Arbeitsspitze des Implantatbettformers herangezogen wird. Bei dieser Ausführungsform sind in weiterer Ausbildung der Erfindung die konischen Konturlinien der Arbeitsspitze des Vorformers in Projektion auf die Arbeitsspitze des Implantatbettformers durch die Innenkanten der Abstufungen der abgestuften Konturlinien der Arbeitsspitze des Implantatbettformers verlaufend ausgebildet. Somit wird bereits mit dem Vorformer die Kieferöffnung soweit vorgeformt, daß mit der abgestuften Arbeitsspitze des Implantatbettformers nur noch die Stufenbereiche zum Einsetzen des Stufenzylinders bzw. der Stufenschraube als Implantat gebildet werden müssen.

Das vordere Ende der Arbeitsspitze des Implantatbettformers ist spitzkegelig, insbesondere unter 120° ausgeformt, um ein leichtes Eindringen des abgestuften Implantatbettformers in die vom Vorformer gebildete Kieferöffnung zu gewährleisten.

In einer weiteren Ausführung der Erfindung weist die Arbeitsspitze des Implantatbettformers zur Bildung einer Kieferöffnung für ein Zylinderimplantat eine konische Außenkontur auf, wobei der Konuswinkel des Implantatbettformers etwas größer ist als der Konuswinkel des Vorformers. Auch hierdurch erfolgt beim Einbringen des konischen Implantatbettformers nach dem Herausziehen des konischen Vorformers eine Verdichtung des Knochenmaterials seitlich der Kieferöffnung. In die so gebildete leicht konische Kieferöffnung können Zylinderimplantate mit vollständigem, teilweisem oder ohne Gewinde sicher eingesetzt werden.

Ferner ist in noch weiterer Ausbildung der Erfindung der unmittelbar an die Arbeitsspitzen von Vorformer und Implantatbettformer anschließende Schaftbereich mit mindestens einer umlaufenden Kerbe als Tiefenskala versehen. Diese ermöglicht dem Operateur die Eindringtiefe des Vorformers und des Implantatbettformers genauestens zu kontrollieren, um diese an die Länge des einzusetzenden Implantats genauestens anzupassen. In bevorzugter Ausführungsform weist der Schaftbereich von Vor- und Implantatbettformer mehrere im Abstand voneinander angeordnete Kerben als Tiefenskala auf.

Beim Einbringen von Schraubenimplantaten in die mittels des Instruments (Osteotom) nach Summers leicht konisch ausgebildete Höhlung besteht die Gefahr,daß es bei dem nun verdichteten und nicht mehr so elastischen Knochen zu unkontrollierten Brüchen und Absplitterungen kommen kann. Durch die ungenügende Berücksichtigung der spezifischen Implantatform kann es schon beim Einbringen des Implantats zur Fraktur der filigranen Knochenanteile kommen, die zum Verlust des Implantatbettes führen kann, zumal sich die Osteotomtechnik bei sehr ungünstigen (schmalen) Kieferkämmen anbietet, wobei der vorhandene Knochen substanzsparend gedehnt wird. Dies trifft besonders bei Schraubenimplantanten zu, da diese nicht wie Zylinderimplantate selbst als Osteotom benutzt und in den Knochen getrieben werden, sondern z.T. maschinell langsam in den Knochen eingedreht werden und z.T. ein vorgeschnittenes Gewinde benötigen, besonders bei dem durch die Osteotomtechnik verdichteten und verhärteten Knochen.

Die Erfindung ist nachfolgend anhand von zwei unterschiedlichen Sätzen von Dentalinstrumenten, jeweils bestehend aus einem Vorformer mit konischer Arbeitsspitze und einem an die Außenkontur des Implantats angepaßten Implantatbettformer näher erläutert. Es zeigen:
- Fig. 1: Einen Vorformer welcher kein Gegenstand, der Erfindung ist
- Fig. 2: den Implantatbettformer in der ersten Ausführungsform,
- Fig. 3: die Arbeitsspitze des Implantatbettformers nach Fig. 2 im Knochenmaterial des Oberkiefers mit Darstellung der Konturlinien des zugehörigen Vorformers gemäß Fig. 1,
- Fig. 4: und 6 den Vorformer gemäß Fig. 1 mit anderen Abmessungen,
- Fig. 5: und 7 die zu den Vorformern nach Fig.4 bzw. 6 gehörenden Implantatbettformer.
- Fig. 8: bis 10 den Vorformer in einer Ausführungsform wobei die Vorformer gemäß den Fig. 9 und 10 kein Gegenstand der Erfindung sind
- Fig. 11 bis 13: den Implantatbettformer

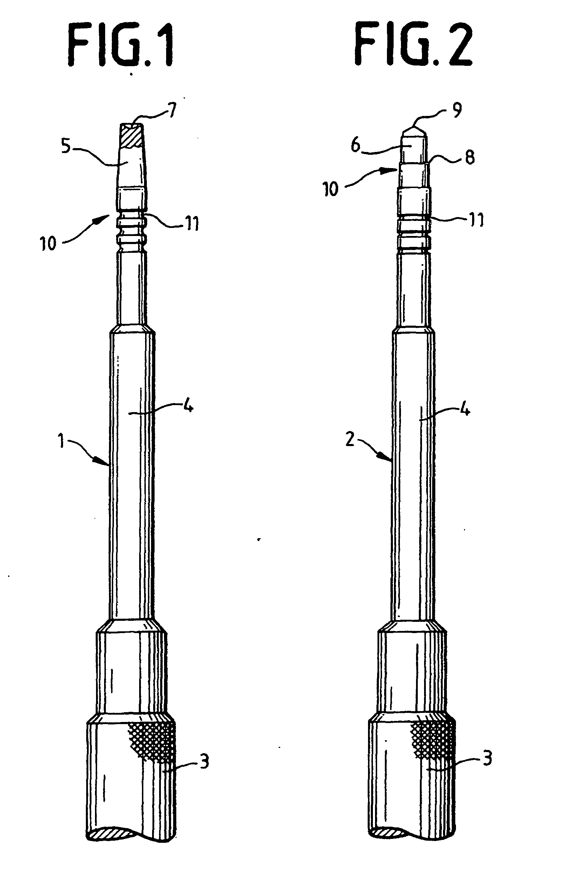

Ein Satz von kieferchirurgischen Instrumenten (Osteotom) zur Schaffung von Kieferöffnungen zum Einsetzen von nicht dargestellten Implantaten umfaßt jeweils mindestens einen Vorformer 1 gemäß Fig. 1 und einen Implantatbettformer 2 genannten Fertigformer gemäß Fig. 2. Diese bestehen jeweils aus einem Halter 3 als Handgriff mit einer Oberfläche aus Kreuzrändel, einem daran anschließenden im Querschnitt kreisrunden, gestuften Schaft 4 und einer Arbeitsspitze 5 beim Vorformer 1 sowie einer Arbeitsspitze 6 beim Implantatbettformer 2. Vorformer 1 und Implantatbettformer 2 bestehen aus hochfestem, rostfreien metallischen Werkstoff, insbesondere V4A. Der die jeweilige Arbeitsspitze 5,6 mit dem jeweiligen Halter 3 verbindende Schaft 4 ist mehrfach abgestuft, um vom Durchmesser von 10 mm des Halters 3 über zwei Stufen von 8 bzw. 5 mm Durchmesser auf den später noch beschrieben werdenden Durchmesser der jeweiligen Arbeitsspitze 5,6 zu kommen.Der den Handgriff bildende Halter 3, der Schaft 4 und die Arbeitsspitzen 5,6 haben je einen kreisförmigen Querschnitt und sind einstückig ausgebildet.

Die Arbeitsspitze 5 des Vorformers 1 gemäß Fig. 1 ist konisch ausgebildet und weist am vorderen, distalen Ende eine konkave Kalotte 7 auf. Die Arbeitsspitze 6 des Implantatbettformers 2 gemäß Fig. 2 umfaßt in Richtung auf das Ende drei Stufen 8 mit jeweils kleineren Durchmessern sowie eine spitzkegelig ausgeformte Spitze 9, deren Kegelwinkel 118° beträgt. Die Stufen 8 sind dabei an die Stufen eines Stufenzylinders oder einer Stufenschraube als Implantat angepaßt, wie es später noch näher ausgeführt werden wird.

Unmittelbar an die Arbeitsspitzen 5,6 von Vorformer 1 und Implantatbettformer 2 sind die anschließenden Schaftbereiche 10 mit mindestens je einer umlaufenden Kerbe 11 als Tiefenskala versehen. Im Ausführungsbeispiel gemäß den Figuren 1 und 2 sind jeweils drei Kerben 11 in bestimmten Abständen zum distalen Ende des Vorformers 1 bzw.des Implantatbettformers 2 angeordnet,

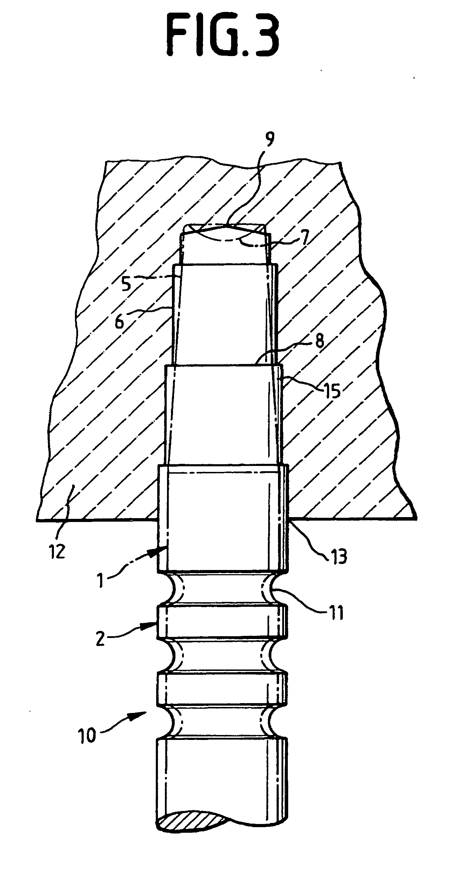

Die Fig. 3 zeigt in schraffierter Darstellung das Knochenmaterial des Oberkiefers 12 eines Patienten, in welchen eine Kieferöffnung 13 eingebracht ist, die entsprechend den Stufen 8 des Implantatbettformers ausgebildet ist. Der Implantatbettformer 2 ist in Fig. 3 mit dicken Linien dargestellt, wohingegen in den Implantatbettformer 2 mit dünnen Linien die Konturen des Vorformers 1 eingezeichnet sind. Dies zeigt, daß die konischen Konturlinien der Arbeitsspitze 5 des Vorformers 1 in der Projektion auf die Arbeitsspitze 6 des Implantatbettformers 2 durch die Innenkanten 14 der Stufen 8 der abgestuften Konturlinien der Arbeitsspitze 6 des Implantatbettformers 2 verlaufen. Dies zeigt, daß mittels der Arbeitsspitze 5 des Vorformers 1 (dünne Linienzüge) zunächst eine konische Kieferöffnung im Oberkiefer 12 geschaffen wird, wobei aufgrund der Kalotte 7 am Ende des Vorformers 1 ein linsenförmiges Knochenmaterial stehen bleibt, das anschließend beim Eindringen des Implantatbettformers 2 durch die Spitze 9 verdrängt und komprimiert wird. Die in der Projektion jeweils seitlichen,dreieckförmigen Knochenmaterialbereiche 15, die außerhalb der Konturlinie der Arbeitsspitze 5 des Vorformers (dünne Linienzüge) im Bereich der Stufen 8 gebildet sind, werden mittels der Stufen 8 des Implantatbettformers 2 bei dessen Eindringen verdrängt.

Die Fig. 3 zeigt auch die sich überdeckenden Kerben 11 von Vorformer 1 und Implantatbettformer 2, wobei aufgrund von Unterschieden im Durchmesser zwischen dem etwas dünneren Vorformer 1 und dem etwas dickeren Implantatbettformer 2 unterschiedliche Innendurchmesser der Kerben 11 ersichtlich sind. Die Kerben 11 dienen dem Dentalmediziner dazu, das jeweilige Dentalinstrument, Vorformer 1 bzw. Implantatbettformer 2, genauestens in der Tiefe des Knochenmaterials des Oberkiefers 12 zu positionieren.

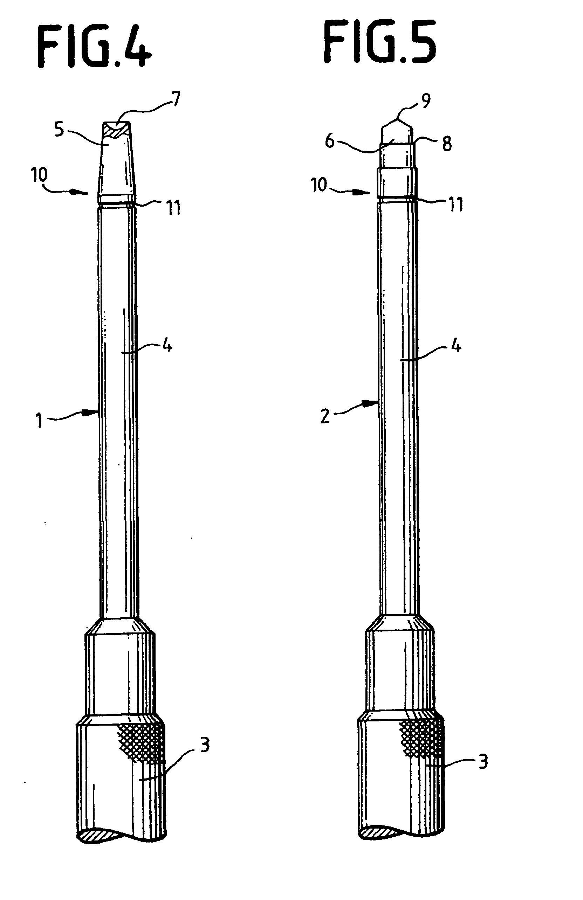

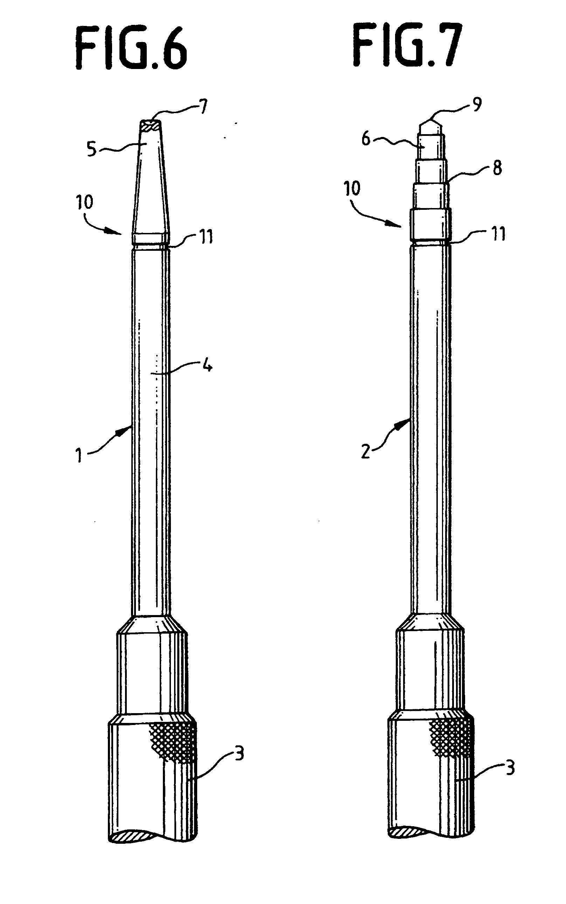

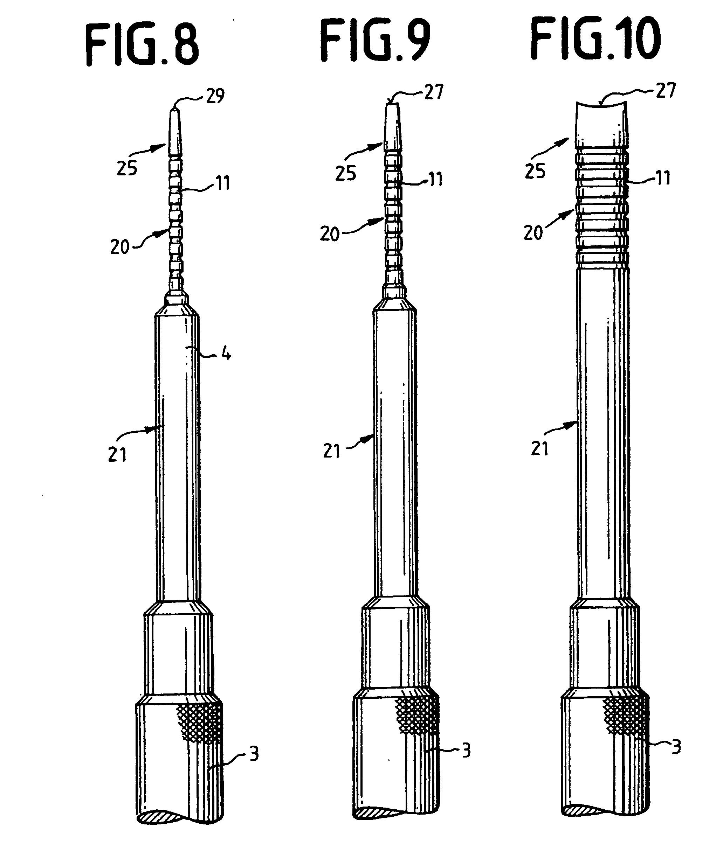

Die Fig. 4 und 6 zeigen abgewandelte Vorformer 1 mit unterschiedlichen Abmessungen der Arbeitsspitzen 5 sowie mit jeweils nur einer einzigen Kerbe 11. Die zugehörigen Implantatbettformer 2 sind in den Fig. 5 bzw. 7 dargestellt. Zu beachten ist, daß der Implantatbettformer 2 gemäß der Ausführungsform in Fig. 7 vier Stufen 8 aufweist, wohingegen die Implantatbettformer 2 gemäß den Figuren 2 und 5 nur jeweils drei Stufen 8 aufweisen.

Der Satz von kieferchirurgischen Instrumenten (Osteotom) gemäß den Fig. 1 bis 7 dient zum Einsetzen von Frialit-2 Stufenzylindern oder Stufenschrauben, d.h. abgestuften Implantaten ohne und mit Außengewinde. Bei einem Implantat mit Außengewinde dient als Maß für die Stufen 8 des Implantatbettformers 2 der jeweilige Gewindeinnendurchmesser.

Ein Satz von Instrumenten dieser Ausführungsform hat folgende Abmessungen:

| Vorformer 1 (mm) | Implantatbettformer 2 | Stufendurchmesser (mm) | Skalierung (mm) |
|---|---|---|---|
| 3,3; 2,5 | 3,8 | 2,6; 3,0; 3,4 | 11;13;15 |
| 4,4; 3,0 | 4,5 | 3,5; 4,0 | 10 |
| 4,4; 2,4 | 4,5 | 3,5; 4,0 | 13 |
| 4,4; 2,2 | 4,5 | 3,0; 3,5; 4,0 | 15 |
| 5,4; 3,4 | 5,5 | 4,2; 4,8; | 10 |
| 5,4; 2,9 | 5,5 | 3,6; 4,2; 4,8 | 13 |
| 5,4; 2,5 | 5,5 | 3,0; 3,5; 4,2; 4,8 | 15 |
| 6,0; 3,85 | 6,5 | 4,6; 5,2; 5,8 | 13 |
| 6,0; 3,85 | 6,5 | 4,6; 5,2; 5,8 | 15 |

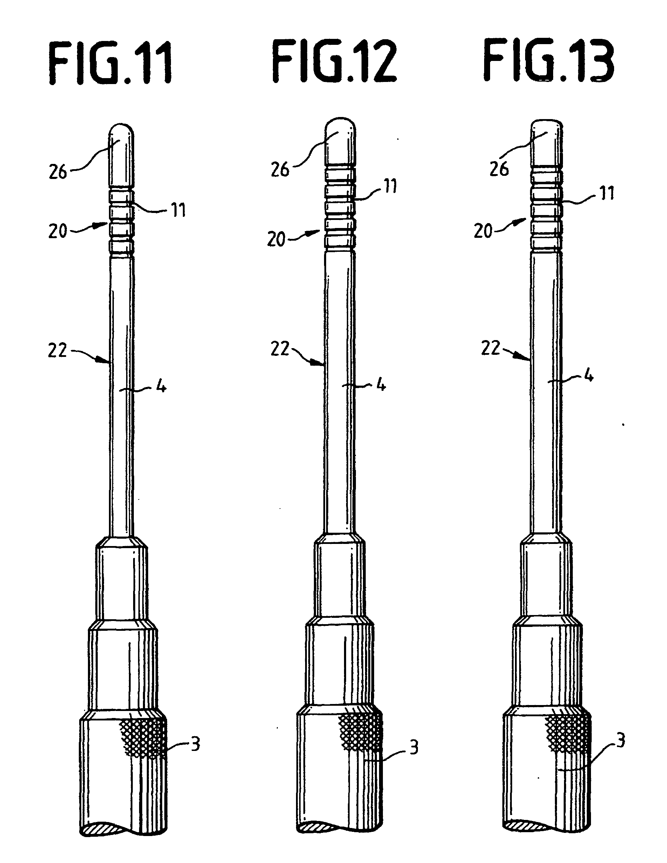

Die in den Fig. 8 bis 13 dargestellte Ausführungsform von kieferchirurgischen Instrumenten (Osteotom) zeigt einen Satz von zehn Vorformern 21 (Fig. 8 bis 10), beginnend mit einer Arbeitsspitze 25 mit einem Spitzendurchmesser von 1 mm, und drei Implantatbettformern 22 (Fig.11 bis 13).

Die Arbeitsspitze 25 des Vorformers 21 gemäß Fig. 8 ist konisch ausgebildet und weist am Ende eine spitzkegelige Spitze 29 vom Kegelwinkel 118° und dem Durchmesser 1 mm auf. Diese Arbeitsspitze 25 hat einen sich konisch erweiternden Schaft vom Durchmesser 1,5 mm. Alle weiteren Vorformer dieses Satzes haben eine konkave Kalotte 27 anm freien Ende und erweitern ihren Durchmesser nur um 0,5 mm. Dieser Instrumentensatz an Vorformern 21 dient zur universellen (Vor-)Erweiterung des Knochens für die gängigen Implantatgrößen.

Der an die Arbeitsspitze 25 anschließende Schaftbereich 20 ist mit acht Kerben 11 mit unterschiedlichen Abständen von der Spitze 29 als Skalierung ausgebildet. Die in den Fig. 9 und 10 dargestellten Vorformer 21 unterschiedlicher Durchmesser weisen entsprechende Kerben 11 in gleichen Abständen wie beim Vorformer 21 nach Fig. 8 auf. Die Arbeitsspitze 25 ist mit einer konischen Außenkontur versehen, deren Konuswinkel etwas größer ist als der Konuswinkel der Arbeitsspitze 25 des Vorformers 21 nach Fig. 8 mit der Spitze 29. Außerdem ist das Ende der Vorformer 21 nach den Fig. 9 und 10 mit einer konkaven Kalotte 27 versehen. Der Satz von Instrumenten (Osteotom) der in den Fig. 8 bis 10 dargestellten Ausführungsform dient zur Bildung und Dehnung der Kieferöffnungen bis zum Durchmesser von 6 mm zur nachfolgenden Schaffung von Öffnungen mittels der Implantabettformer 22 nach Fig. 11 bis 13. Die Abmessungen der Vorformer 21 sind:
- Vorformer 21 (mit Spitze): 1,5 mm; 1,0 m
- Vorformer 21 (mit Kalotte): 2,0 mm; 1,5 mm
2,5 mm; 2,0 mm
3,0 mm; 2,5 mm
3,5 mm; 3,0 mm
4,0 mm; 3,5 mm
4,5 mm; 4,0 mm
5,0 mm, 4,5 mm
5,5 mm ; 5,0 mm
6,0 mm; 5,5 mm

Die Länge der konischen Arbeitsspitzen 25 beträgt jeweils 6,0mm.

Die acht Kerben 11 haben vom Ende Abstände von 6,0 mm bis 20,0 mm .

Die in den Figuren 11 und 13 dargestellten Implantatbettformer 22 der zweiten Ausführungsform des Satzes von kieferchirurgischen Instrumenten (Osteotom) umfaßt drei Implantatbettformer 22 mit zylindrischen Arbeitsspitzen und kreisförmigen oder flach abgerundeten Enden sowie einer Anzahl von Kerben 11 zur Bildung von Skalen. Die Implantatbettformer 22 dienen zum Einsetzen von Zylinderimplantaten ohne Gewinde oder mit teilweise oder ganz durchgängigen Gewinde. Die Abmessungen der zylindrischen Implantatbettformer 22 vom Typ ITI-Stramann-Bonefit sind:

Arbeitsspitzendurchmesser 2,7 mm oder 3,5 mm. Länge der Arbeitsspitzen 6,0 mm oder 8,0 mm.

Die fünf bzw. sechs Kerben 11 haben vom Arbeitsende Abstände von 6,0 mm bzw. 8,0 mm .

## Patentansprüche

1. Satz von kieferchirurgischen Instrumenten Osteotome, aus Halter, Schaft und im Querschnitt kreisförmiger Arbeitsspitze zur Schaffung von Öffnungen im Kieferknochen zum Einsetzen von Implantaten,
**gekennzeichnet durch**
mindestens einen Vorformer (21) mit konischer Arbeitsspitze (5; 25), deren vorderes Ende spitzkegelig mit einer Spitze ausgeformt ist,
und mindestens einen Implantatbettformer (2; 22), dessen Arbeitsspitze (6;26) entweder am vorderen Ende spitzkegelig oder flach abgerundet ausgebildet ist und eine zum freien Ende hin im Durchmesser abgestufte Aussenkontur oder eine zylindrische oder eine konische Aussenkontur aufweist,
wobei der Durchmesser der Arbeitsspitze (5; 25) des Vorformers (21) etwas kleiner ist als der größte Durchmesser der Arbeitsspitze (6;26) des Implantatbettformers (2; 22).

2. Satz nach Anspruch 1, **dadurch gekennzeichnet,**
**daß** die konischen Konturlinien der Arbeitsspitze (5) des Vorformers (21) in der Projektion auf die abgestufte Arbeitsspitze (6) des Implantatbettformers (2) durch die Innenkanten (14) der Stufen (8) der abgestuften Konturlinien der Arbeitsspitze (6) des Implantatbettformers (2) verlaufen.

3. Satz nach Anspruch 1, **dadurch gekennzeichnet, daß** der Kegelwinkel der Spitze (9) etwa 120° beträgt.

4. Satz nach einem der Ansprüche 1 bis 3, **dadurch gekennzeichnet, daß** der Konuswinkel des Implantatbettformers (2) etwas grösser ist als der Konuswinkel des Vorformers (1).

5. Satz nach einem der Ansprüche 1 bis 4, **dadurch gekennzeichnet, daß** der unmittelbar an die Arbeitsspitzen von Vorformer (21) und Implantatbettformer (2) anschließende Schaftbereich (15) mit mindestens einer umlaufenden Kerbe (8) als Tiefenskala versehen ist.

6. Satz nach Anspruch 5, **dadurch gekennzeichnet, daß** der Schaftbereich (15) mehrere im Abstand voneinander angeordnete Kerben (8) als Tiefenskala aufweist.

## Claims

1. A kit of dental surgical instruments (osteotomes), comprising a handpiece, a shaft and a working tip having a circular cross section for making holes in the jaw for the placing of implants,
**characterized by**
at least one pre-former (21) having a conical working tip (5; 25), the front end of which forms a pointed cone with a tip,
and at least one implant socket former (2; 22), the working tip (6; 26) of which is at the front end either a pointed cone or flatly rounded and has an outer contour with a stepped diameter towards the free end or a cylindrical or conical outer contour,
the diameter of the working tip (5; 25) of the pre-former (21) being slightly smaller than the largest diameter of the working tip (6; 26) of the implant socket former (2; 22).

2. A kit according to claim 1, **characterized by** that the conical contour lines of the working tip (5) of the pre-former (21) extend in the projection on the stepped working tip (6) of the implant socket former (2) through the inner edges (14) of the steps (8) of the stepped contour lines of the working tip (6) of the implant socket former (2).

3. A kit according to claim 1, **characterized by** that the cone angle of the tip (9) is approximately 120°.

4. A kit according to one of claims 1 to 3, **characterized by** that the cone angle of the implant socket former (2) is slightly larger than the cone angle of the pre-former (21).

5. A kit according to one of claims 1 to 4, **characterized by** that shaft section (15) immediately following the working tips of pre-former (21) and implant socket former (2) is provided with at least one circumferential notch (8) as a depth scale.

6. A kit according to claim 5, **characterized by** that the shaft section (15) comprises several spaced notches (8) as a depth scale.

## Revendications

1. Jeu d'instruments chirurgicaux (ostéotomes), comprenant une poignée, une tige et une pointe de travail ayant une section transversale circulaire pour la réalisation de cavités dans la mâchoire pour la pose d'implants,
caractérisé en
au moins un dispositif de préformation (21) ayant une pointe de travail (5; 25) conique, dont l'extrémité avant est un cône de petit angle avec une pointe,
et au moins un dispositif de formation du lit de l'implant (2; 22), dont la pointe de travail (6; 26) est à l'extrémité avant un cône de petit angle ou platement arrondie et comprend un contour extérieur d'un diamètre en étapes vers l'extrémité libre ou un contour extérieur cylindrique ou conique,
le diamètre de la pointe de travail (5; 25) du dispositif de préformation (21) étant légèrement inférieur au diamètre le plus grand de la pointe de travail (6; 26) du dispositif de formation du lit de l'implant (2; 22).

2. Jeu selon la revendication 1, **caractérisé en ce que** les lignes de contour coniques de la pointe de travail (5) du dispositif de préformation (21) s'étendent dans la projection sur la pointe de travail (6) en étapes du dispositif de formation du lit de l'implant (2) à travers des arêtes intérieures (14) des étapes (8) des lignes de contour en étapes de la pointe de travail (6) du dispositif de formation du lit de l'implant (2).

3. Jeu selon la revendication 1, **caractérisé en ce que** l'angle du cône de la pointe (9) est d'environ 120°.

4. Jeu selon une des revendications 1 à 3, **caractérisé en ce que** l'angle du cône du dispositif de formation du lit de l'implant (2) est légèrement supérieur à l'angle du cône du dispositif de préformation (21).

5. Jeu selon une des revendications 1 à 4, **caractérisé en ce que** la section (15) de la tige suivant immédiatement aux pointes de travail du dispositif de préformation (21) et du dispositif de formation du lit de l'implant (2) est pourvue d'au moins une encoche circonférentielle (8) comme échelle de profondeur.

6. Jeu selon la revendication 5, **caractérisé en ce que** la section (15) de la tige comprend plusieurs encoches (8) à distances comme échelle de profondeur.
